# EUROPÄISCHE PATENTSCHRIFT

(11) **EP 0 884 024 B1**
(45) Veröffentlichungstag und Bekanntmachung des Hinweises auf die Patenterteilung: **13.08.2003**
(21) Anmeldenummer: 97810373.7
(22) Anmeldetag: 13.06.1997
(51) Int. Cl.: A61B 18/14

(54) **Ablationskatheter mit mehreren Polen**
Multipolar ablation catheter
Cathéter multipolaire d'ablation

(43) Veröffentlichungstag der Anmeldung: 16.12.1998
(73) Patentinhaber: Sulzer Osypka GmbH, 79639 Grenzach-Wyhlen (DE)
(72) Erfinder: Geistert, Wolfgang, Dr., 79618 Rheinfelden-Herten (DE)
(74) Vertreter: Maucher, Wolfgang, Dipl.-Ing.

(56) Entgegenhaltungen:
- WO-A-95/10225
- US-A- 5 083 565

## Beschreibung

Die Erfindung betrifft einen Ablationskatheter mit mehreren Polen Anspruch 1 gemäß.

Das Dokument WO-A-095/10225 offenbart eine Vorrichtung gemäß Oberbegriff des Anspruchs 1.

Derartige Ablationskatheter werden zu diagnostischen und therapeutischen Zwecken verwendet. Einerseits sind mit den Polen herzeigene Signale, nämlich EKG-Signale, ableitbar (Diagnose); andererseits sind Ablationen mittels HF-Energieabgaben durchführbar (Therapie). Bei der Ablation werden mit Vorteil mehrere Pole zusammengeschaltet, um grössere Läsionen zu erzielen. Dazu werden Schalter benutzt. Nach dem Schliessen der Schalter ist es jedoch nicht mehr möglich, ein EKG-Signal zwischen Polen, die durch die Schalter kurzgeschlossen sind, abzuleiten.

Bei bestimmten Herzrhythmusstörungen ist es erforderlich, intrakardiale Elektrogramme in Gebieten genau zu erfassen, in denen Arrhythmien ausgelöst werden. Um die Quelle der Arrhythmie genau detektieren zu können, werden Katheter mit Polen ausgestattet, die meist dicht beieinander liegen. Nach erfolgter Detektierung werden die zur Diagnose verwendeten Pole gleich zur Therapie benutzt, indem zwecks thermischer Zerstörung HF-Energie an das Gewebe abgegeben wird. Um grössere Läsionen (z.B. linien- oder flächenförmige) zu erzeugen, werden die Pole oder ein Teil der Pole durch Schliessen von Schaltern kurzgeschlossen. Um den Behandlungserfolg anhand von EKG-Messungen kontrollieren zu können, müssen die Schalter wieder geöffnet werden.

Es ist Aufgabe der Erfindung, einen Ablationskatheter zu schaffen, der auf einfache Weise Messungen von intrakardialen Elektrogrammen (EKG-Ableitung) wie auch gleichzeitige HF-Energieabgaben über mehrere Pole erlaubt. Diese Aufgabe wird durch den in Anspruch 1 definierten Gegenstand gelöst. Die abhängigen Ansprüche beziehen sich auf vorteilhafte Ausführungsformen.

Der erfindungsgemässe Ablationskatheter mit mehreren Polen wird zur intrakardialen Behandlung von Herzgewebe verwendet. Er weist Anschlüsse zur elektrischen Verbindung mit einem HF-Generator sowie mit einem EKG-Messgerät auf. Mindestens zwei Pole sind über einen Kondensator verbunden. Dieser Kondensator ist so ausgelegt, dass gleichzeitig eine HF-Energieabgabe über die beiden Pole und eine EKG-Ableitung zwischen den zwei Polen möglich ist.

Erfindungsgemäss sind zwischen Polen, die für die EKG-Ableitung und die HF-Energieabgabe verwendet werden, Kondensatoren geschaltet. Deren Kapazität wird so bemessen, dass die Kondensatoren einerseits dem an das Gewebe weiterzuleitenden HF-Strom einen nur unerheblichen Blindwiderstand entgegensetzen und andererseits aber bei niedrigen Frequenzen einen genügend hohen Widerstand erzeugen, so dass die EKG-Ableitung nicht wesentlich beeinflusst wird.

Bei der EKG-Ableitung ist der zwischen den Polen bestehende Gewebewiderstand zum Kondensator und zum Eingangswiderstand des EKG-Messgeräts parallel geschaltet. Der Gewebewiderstand hängt unter anderem von der Grösse der Pole ab; er liegt in der Grössenordnung von 100 bis 300 Ohm. Der genannte Eingangswiderstand ist in der Regel mindestens um drei Zehnerpotenzen grösser und muss daher bezüglich der Auslegung des Kondensators nicht berücksichtigt werden. Der Blindwiderstand des Kondensators muss bei EKG-Frequenzen, d.h. bis rund 500 Hz, viel grösser als der Widerstand der Signalquelle sein, wobei dieser Widerstand der Gewebewiderstand ist. Ein Kondensator mit der Kapazität von beispielsweise 33 nF hat bei 500 Hz einen Blindwiderstand von rund 10⁴ Ohm, liegt also mehr als eine Zehnerpotenz über dem Gewebewiderstand und hat daher einen akzeptablen Wert.

Der HF-Strom mit einer Frequenz von rund 500 kHz wird mittels mindestens eines Katheterpols und einer Gegenelektrode in das Gewebe eingespeist. Für das zwischen dem Katheterpol und der Gegenelektrode liegende Gewebe kann ein Widerstand (ebenfalls abhängig von der Polgrösse) von typischerweise 100 Ohm angenommen werden. Der zwischen dem HF-Generator und dem Katheterpol in Serie geschaltete Kondensator (33 nF) hat bei der HF-Frequenz einen Blindwiderstand von rund 10 Ohm, ist also etwa eine Grössenordnung kleiner als der Gewebewiderstand. Da es sich um einen Blindwiderstand handelt, muss der HF-Generator nur weniges mehr an Leistung erzeugen, um die gleiche Energie an das Gewebe zu übertragen wie ohne den in Serie geschalteten Kondensator.

Nachfolgend wird die Erfindung anhand der Zeichnungen näher erläutert. Es zeigen:
- Fig. 1: ein Schaltschema für eine erfindungsgemässe Ablationsanordnung, deren Katheter zwei Pole aufweist,
- Fig. 2: ein Schaltschema für eine zweite Ausführungsform eines erfindungsgemässen Ablationskatheters,
- Fig. 3: das distale Ende eines Katheters mit zwei Polen,
- Fig. 4: das distale Ende eines Katheters mit vier Polen und
- Fig. 5: einen Ausschnitt eines Katheters, bei dem Pole in einem Bereich angeordnet sind, der sich nicht am distalen Ende befindet.

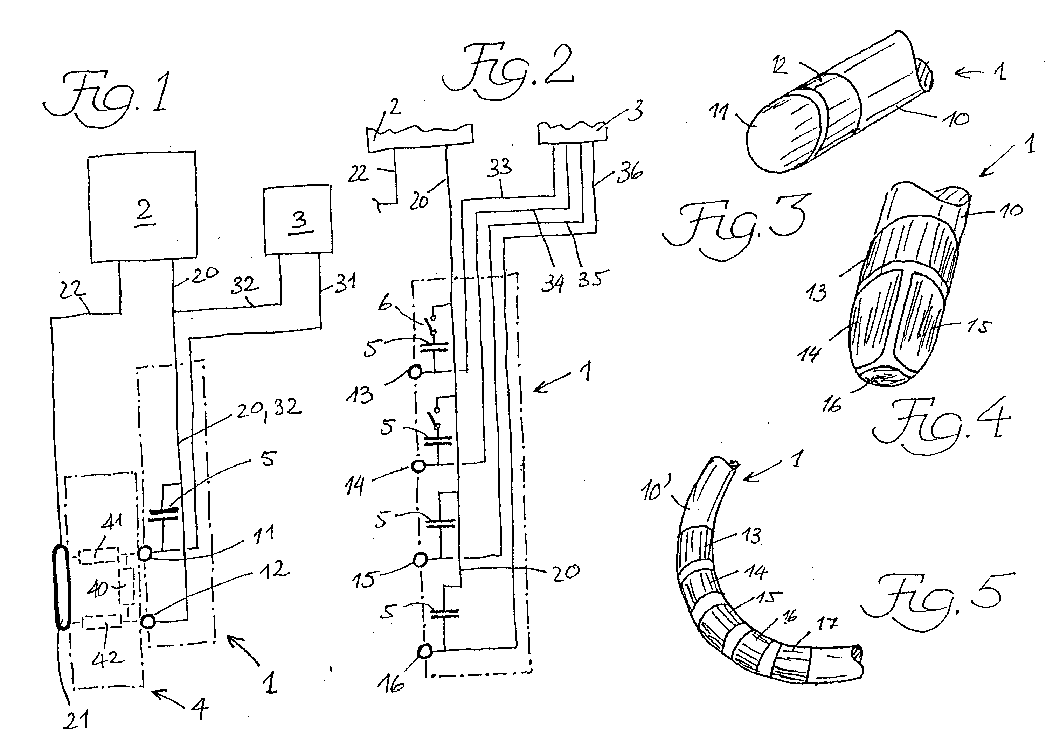

Die Ablationsanordnung gemäss Fig.1 umfasst neben einem erfindungsgemässen Ablationskatheter 1 (strichpunktiertes Rechteck) einen HF-Generator 2, der über eine Leitung 20 mit dem Katheter 1 und über eine Leitung 22 mit einer Gegenelektrode 21 verbunden ist, sowie ein EKG-Messgerät 3, das über Leitungen 31 und 32 mit Polen 11 bzw. 12 des Katheters 1 in Verbindung steht. Zwischen den Polen 11 sowie 12 und der Gegenelektrode 21 befindet sich ein zu behandelndes Gewebe 4 (strichpunktiertes Rechteck). Hinsichtlich der EKG-Ableitung wirkt zwischen den Polen 11 und 12 ein Gewebewiderstand 40; hinsichtlich der HF-Energieabgabe wirken zwischen den Polen 11 und 12 einerseits und der Gegenelektrode 21 andererseits die Gewebewiderstände 41 und 42. Wie bereits oben erwähnt gelten für die Widerstände grössenordnungsmässig folgende Werte: 100 - 300 Ohm für den Widerstand 40, 100 Ohm jeweils für die Widerstände 41 und 42.

Erfindungsgemäss ist zwischen den Polen 11 und 12 ein Kondensator 5 angeordnet, für dessen Kapazität ein Wert von beispielsweise 33 nF vorzusehen ist (siehe die oben gemachten Überlegungen). Die Verbindung 32 des Pols 12 zum EKG-Messgerät 3 und die Verbindung 20, über die der Pol 12 direkt mit dem HF-Generator 2 verbunden ist, werden teilweise über ein gemeinsames Leitungsstück hergestellt. Der andere Pol 11 ist über den Kondensator 5, der für den HF-Strom durchlässig ist, und ebenfalls über die Leitung 20 mit dem HF-Generator 2 verbunden, so dass eine HF-Energieabgabe gleichzeitig über beide Pole 11 und 12 erfolgen kann. Bei den tieferen Frequenzen des EKG-Signals trennt der Kondensator 5 die Pole 11 und 12 weitgehend, so dass gleichzeitig mit der HF-Energieabgabe auch intrakardiale Elektrogramme durch das EKG-Messgerät 3 aufgenommen werden können.

Bei dem in Fig.2 dargestellten Katheter 1 sind alle Pole - nämlich vier Pole 13, 14, 15 und 16 - über Kondensatoren 5 mit der separaten HF-Zuleitung 20 verbunden, die ausserhalb des Katheters 1 an den HF-Generator 2 angeschlossen ist. Ausserdem sind die Pole 13 bis 16 über Polzuleitungen 33, 34, 35 und 36 mit dem EKG-Messgerät verbunden.

Bei dem Ausführungsbeispiel nach Fig.2 ist jedem für EKG-Ableitungen sowie HF-Energieabgaben vorgesehenen Pol 13 bis 16 ein Kondensator 5 gemäss der Erfindung zugeordnet, und jeder Kondensator 5 ist an eine zu den Polzuleitungen 33 bis 36 separat geführte HF-Zuleitung 20 angeschlossen. Es wäre auch möglich, dass nur bis auf Ausnahme eines Pols jedem für EKG-Ableitungen sowie HF-Energieabgaben vorgesehenen Pol ein Kondensator 5 zugeordnet ist, und der ausgenommene Pol direkt an den HF-Generator sowie an das EKG-Messgerät angeschlossen ist. Dies ist beim ersten Ausführungsbeispiel der Fall, wo der Pol 12 diese Ausnahme bildet.

Das zweite Ausführungsbeispiel hat gegenüber dem ersten den Vorteil, dass sich die Pole 13 bis 16 symmetrisch verhalten und dass die für die EKG-Ableitung wirksame Koppelkapazität zwischen den Polen durch Serieschaltungen von jeweils zwei Kondensatoren 5 halbiert wird.

Es können auch Schalter 6 - siehe Fig.2 - vorgesehen sein, mit denen zu Beginn oder während der Behandlung gewählt werden kann, über welche Pole eine HF-Abgabe erfolgen soll.

Selbstverständlich kann der Katheter 1 auch noch weitere Pole aufweisen, die nicht in die HF-Abgabe einbezogen sind und denen daher auch keine Kondensatoren 5 zugeordnet sind.

In der Regel sind die Pole an dem distalen Ende 10 des Katheters 1 oder in der Nähe dieses Endes 10 angeordnet, wie es in Fig.3 ein zweipoliger Katheter 1 mit einer quergeteilten Spitze zeigt oder in Fig.4 ein vierpoliger Katheter 1, der eine dreifach längsgeteilte Spitze und einen dahinter angeordneten vierten Pol aufweist. Das Ausführungsbeispiel der Fig.5 stellt einen Katheter 1 dar, bei dem sechs Pole 13 bis 17 in einem nicht distalen Bereich 10' entlang des Katheters angeordnet sind. Es sind natürlich auch viele andere Polanordnungen denk- und ausführbar.

Die Kondensatoren 5 sind in den Katheter 1 eingebaut. Sie können insbesondere in einem nicht dargestellten Griff des Katheters angeordnet sein.

Mit dem erfindungsgemässen Gerät kann während oder unmittelbar nach der Ablation der Behandlungserfolg sofort kontrolliert werden. Es sind wärend der Durchführung der Ablation keine Betätigungen von Schaltern nötig, so dass das Gerät sich sehr einfach handhaben lässt.

## Patentansprüche

1. Ablationskatheter (1) mit mehreren Polen (11, 12, 13, 14, 15, 16, 17), zur intrakardialen Behandlung von Herzgewebe, mit elektrischen Anschlüssen (20, 31, 32, 33, 34, 35, 36) an einen HF-Generator (2) sowie an ein EKG-Messgerät (3),
**dadurch gekennzeichnet, dass** mindestens zwei Pole (11, 12) über einen Kondensator (5) verbunden sind, der in den Ablationskatheter eingebant ist und der so ausgelegt ist, dass gleichzeitig eine HF-Energieabgabe über die beiden Pole und eine EKG-Ableitung zwischen den zwei Polen möglich ist.

2. Ablationskatheter nach Anspruch 1, **dadurch gekennzeichnet, dass** jedem für EKG-Ableitungen sowie HF-Energieabgaben vorgesehenen Pol (11, 12, 13, 14, 15, 16) ein Kondensator (5) der genannten Art zugeordnet ist.

3. Ablationskatheter nach Anspruch 1, **dadurch gekennzeichnet, dass** bis auf Ausnahme eines Pols (12) jedem für EKG-Ableitungen sowie HF-Energieabgaben vorgesehenen Pol (11) ein Kondensator (5) der genannten Art zugeordnet ist und der ausgenommene Pol (12) direkt an den HF-Generator (2) sowie an das EKG-Messgerät (3) anschliessbar ist.

4. Ablationskatheter nach einem der Ansprüche 1 bis 3, **dadurch gekennzeichnet, dass** die Pole (11, 12, 13, 14, 15, 16) an seinem distalen Ende (10) oder in der Nähe dieses Endes angeordnet sind.

5. Ablationskatheter nach einem der Ansprüche 1 bis 4, **dadurch gekennzeichnet, dass** mehr als zwei Pole (13, 14, 15, 16) vorliegen und dass die Verbindung (20) von mindestens einem der Pole (13, 14) zum HF-Generator (2) durch einen Schalter (6) unterbrechbar ist.

6. Ablationsanordnung mit einem Ablationskatheter (1) gemäss einem der Ansprüche 1 bis 5 und mit einem HF-Generator (2) sowie einem EKG-Messgerät (3).

7. Ablationsanordnung nach Anspruch 6, **dadurch gekennzeichnet, dass** die Kondensatoren (5) in einem Griff des Katheters (1) eingebaut sind.

## Claims

1. Ablation catheter (1), having a plurality of poles (11, 12, 13, 14, 15, 16, 17), for the intracardial treatment of heart tissue, and having electrical connections (20, 31, 32, 33, 34, 35, 36) to an HF generator (2) and to an ECG measuring apparatus (3), **characterised in that** at least two poles (11, 12) are connected via a capacitor (5), which is incorporated in the ablation catheter and is so designed that an HF energy release via the two poles and an ECG leakage between the two poles are simultaneously possible.

2. Ablation catheter according to claim 1, **characterised in that** each pole (11, 12, 13, 14, 15, 16), which is provided for ECG leakages and HF energy releases, has associated therewith a capacitor (5) of the aforesaid type.

3. Ablation catheter according to claim 1, **characterised in that**, with the exception of one pole (12), each pole (11), which is provided for ECG leakages and HF energy releases, has associated therewith a capacitor (5) of the aforesaid type, and the excluded pole (12) is connectable directly to the HF generator (2) and the ECG measuring apparatus (3).

4. Ablation catheter according to one of claims 1 to 3, **characterised in that** the poles (11, 12, 13, 14, 15, 16) are disposed at its distal end (10) or in the vicinity of this end.

5. Ablation catheter according to one of claims 1 to 4, **characterised in that** more than two poles (13, 14, 15, 16) are provided, and **in that** the connection (20) between at least one of the poles (13, 14) and the HF generator (2) can be interrupted by a switch (6).

6. Ablation arrangement having an ablation catheter (1) according to one of claims 1 to 5 as well as an HF generator (2) and an ECG measuring apparatus (3).

7. Ablation arrangement according to claim 6, **characterised in that** the capacitors (5) are incorporated in a handle of the catheter (1).

## Revendications

1. Cathéter d'ablation (1) à plusieurs pôles (11, 12, 13, 14, 15, 16, 17) pour le traitement intracardiaque du tissu cardiaque, avec des connexions électriques (20, 31, 32, 33, 34, 35, 36) à un générateur HF (2) ainsi qu'à un électrocardiomètre (3),
**caractérisé par le fait qu'**au moins deux pôles (11, 12) sont reliés par un condensateur (5), intégré dans le cathéter d'ablation, qui est conçu de manière à permettre en même temps une livraison d'énergie HF par les deux pôles et une mesure d'ECG entre les deux pôles.

2. Cathéter d'ablation selon la revendication 1, **caractérisé par le fait qu'**un condensateur (5) du type mentionné est associé à chaque pôle (11, 12, 13, 14, 15, 16) prévu pour des mesures d'ECG ainsi que des livraisons d'énergie HF.

3. Cathéter d'ablation selon la revendication 1, **caractérisé par le fait qu'**un condensateur (5) du type mentionné est associé à chaque pôle (11) prévu pour des mesures d'ECG ainsi que des livraisons d'énergie HF, à l'exception d'un pôle (12) qui peut être relié directement au générateur HF (2) ainsi qu'à l'électrocardiomètre (3).

4. Cathéter d'ablation selon l'une des revendications 1 à 3, **caractérisé par le fait que** les pôles (11, 12, 13, 14, 15, 16) sont disposés à son extrémité distale (10) ou à proximité de cette extrémité.

5. Cathéter d'ablation selon l'une des revendications 1 à 4, **caractérisé par le fait qu'**il existe plus de deux pôles (13, 14, 15, 16) et que la liaison (20) d'au moins un des pôles (13, 14) avec le générateur HF (2) peut être interrompue par un commutateur (6).

6. Dispositif d'ablation comprenant un cathéter d'ablation (1) selon l'une des revendications 1 à 5 et un générateur HF (2) ainsi qu'un électrocardiomètre (3).

7. Dispositif d'ablation selon la revendication 6, **caractérisé par le fait que** les condensateurs (5) sont intégrés dans une poignée du cathéter (1).
